# FASCICULE DE BREVET EUROPEEN

(11) **EP 0 815 848 B1**
(45) Date de publication et mention de la délivrance du brevet: **26.01.2000**
(21) Numéro de dépôt: 97401284.1
(22) Date de dépôt: 06.06.1997
(51) Int. Cl.: A61K 7/48, A61K 7/06

(54) **Utilisation en cosmétique de copolyméres à squelette flexible, greffés par des macromonoméres hydrophobes et rigides ; compositions mises en oeuvre**
Verwendung in der Kosmetik von Kopolymeren mit einer flexiblen Hauptkette, gepfropft mit hydrophoben und Hartmakromonomeren; Zusammensetzungen zu deren Verwendung
Use in cosmetics of copolymers with a flexible backbone, grafted with hydrophobic and rigid macromonomers; compositions using same

(30) Priorité: 28.06.1996 FR 9608113
(43) Date de publication de la demande: 07.01.1998
(73) Titulaire: L'OREAL, 75008 Paris (FR)
(72) Inventeur: Mougin, Nathalie, 75011 Paris (FR); Lion, Bertrand, 93190 Livry Gargan (FR); Mondet, Jean, 93600 Aulnay Sous Bois (FR)
(74) Mandataire: Miszputen, Laurent

(56) Documents cités:
- WO-A-91/15185
- WO-A-91/15186
- WO-A-91/15187
- WO-A-95/01383
- WO-A-96/00562
- WEWERS D: "Modification of organic polymers by reactive functional polydimethylsiloxanes", Paper given at Chem Spec Europe 88, Frankfurt, March 1988, pages 1-2
- LAPP A ET AL.: "Caractérisation et étude physicochimique du polydiméthylsiloxane en bon solvant", MAKROMOL. CHEM., 1985, Vol. 186, page 1919

## Description

La présente invention a trait à l'utilisation de copolymères à squelette flexible, obtenus par polymérisation radicalaire ou polycondensation et greffés par des macromonomères, rigides et hydrophobes dans et pour la préparation de compositions cosmétiques ou dermatologiques ainsi que les compositions mises en oeuvre.

Pour de nombreuses applications cosmétiques, notamment celles destinées au traitement et au soin des cheveux, de la peau ou des cils, on utilise des polymères susceptibles, après application sur le support à traiter et séchage, de former un dépôt ayant des propriétés mécaniques et des propriétés filmogènes. On recherche dans cette optique des polymères à caractère hydrophile pour s'éliminer facilement sous l'action d'une solution aqueuse de tensioactifs. On recherche parallèlement à obtenir un dépôt hydrophobe en surface pour, d'une part, résister à l'humidité environnante notamment un dépôt non-hydroscopique au toucher, un dépôt résistant à la pluie (applications capillaires ou pour la peau) ou un dépôt résistant au liquide lacrymal (mascaras). D'autre part, on recherche un dépôt hydrophobe pour apporter des propriétés cosmétiques telles que la douceur au toucher généralement conférés par les substances hydrophobes en cosmétique.

Pour réunir toutes ces caractéristiques, on mélange en général des polymères d'hydrophilie et d'hydrophobicité différentes ou bien on associe à un polymère hydrophile une substance hydrophobe. Il est généralement difficile de contrôler parfaitement la stratification de ces mélanges après séchage du dépôt pour obtenir à la fois de bonnes propriétés mécaniques et filmogènes et une surface hydrophobe.

Un autre problème relatif aux polymères filmogènes se pose dans le domaine des vernis à ongles aqueux. En effet, il est souvent difficile d'adapter les propriétés du polymère devant se filmifier rapidement à température ambiante et donner un film présentant à la fois une flexibilité importante, une bonne résistance à l'eau tout en conservant une dureté de surface suffisante.

WO 96 00562 A (PROCTER & GAMBLE), 11 janvier 1996, est relatif à une composition cosmétique comprenant un copolymère greffé élastomère et thermoplastique, dont le squelette a une température de transition vitreuse inférieure à 0°C et dont les greffons sont hydrophobes et ont une température de transition vitreuse supérieure à 25°C. WO 91 15186 A (PROCTER & GAMBLE), 17 octobre 1991, et WO 91 15185 A (PROCTER & GAMBLE), 17 octobre 1991, décrivent des compositions de conditionnement des cheveux ou des shampooings, comprenant un polymère greffé par un polydiméthylsiloxane.

L'un des objectifs de la présente invention est donc d'utiliser dans des compositions cosmétiques ou dermatologiques des polymères ayant des propriétés filmogènes satisfaisantes, qui soient hydrophobes en surface et résistants à l'humidité environnante et apportant de bonnes propriétés cosmétiques telles que la douceur au toucher.

Un autre objectif de l'invention est d'utiliser dans et pour la préparation de vernis à ongles aqueux des polymères filmogènes produisant un film ayant à la fois des caractéristiques de flexibilité importantes, résistant bien à l'eau et ayant une dureté de surface satisfaisante.

La Demanderesse a découvert de manière surprenante que ces objectifs pouvaient être atteints en utilisant dans et pour la préparation de compositions cosmétiques ou dermatologiques, des copolymères greffés particuliers dont le squelette est flexible et constitué d'un copolymère susceptible d'être obtenu par polymérisation radicalaire ou par polycondensation et comportant sur la chaîne du squelette au moins un greffon macromonomère rigide et hydrophobe.

La Demanderesse a découvert par ailleurs que les copolymères greffés particuliers de l'invention permettaient la réalisation de gels aqueux présentant d'excellentes propriétés mécaniques et filmogènes et destinés à l'application sur les cheveux, la peau ou les lèvres.

En effet, les copolymères greffés particuliers de l'invention, en particulier ceux présentant un squelette soluble à l'eau et des greffons hydrophobes rigides, pouvaient au contact d'un milieu aqueux conduire au dépôt d'un gel aqueux, doux et non-collant au toucher, se gonflant d'eau et présentant d'excellentes propriétés mécaniques et filmogènes. Ils peuvent notamment être utilisés dans et pour la préparation de produits capillaires pour la fixation des cheveux conservant leurs propriétés fixantes dans un environnement humide. Ils peuvent également être utilisés dans et pour la préparation de produits hydratants pour le soin ou le maquillage de la peau ou des lèvres pouvant contenir des actifs sans conduire à un dépôt collant.

La présente invention a pour objet l'utilisation dans et pour la préparation de compositions cosmétiques ou dermatologiques, d'un copolymère greffé dont le squelette (S) est constitué d'un copolymère de température de transition vitreuse Tg allant de 0 à 30°C, susceptible d'être obtenu par polymérisation radicalaire ou par polycondensation et comportant sur la chaîne du squelette (S) au moins un greffon constitué d'un macromonomère (M) hydrophobe et de température de transition vitreuse T'g supérieure à 25°C.

La présente invention concerne également des compositions cosmétiques ou dermatologiques contenant dans un milieu cosmétiquement acceptable au moins un copolymère greffé dont le squelette (S) est constitué d'un copolymère de température de transition vitreuse Tg allant de 0 à 30°C, susceptible d'être obtenu par polymérisation radicalaire ou par polycondensation et comportant sur la chaîne du squelette (S) au moins un greffon constitué d'un macromonomère (M) hydrophobe et de température de transition vitreuse T'g supérieure à 25°C.

D'autres objets apparaîtront à la lumière de la description et des exemples qui suivent.

Les copolymères de l'invention sont des copolymères greffés solubles ou dispersibles dans l'eau, les alcools inférieurs (en C₁-C₄) ou les mélanges d'eau et d'alcool(s) inférieur(s).

On entend par «greffon hydrophobe» dans tout le texte de la description, tout greffon insoluble dans l'eau, les alcools inférieurs (en C₁-C₄) ou les mélanges d'eau et d'alcool(s) inférieur(s).

On entend par «macromonomère» dans tout le texte de la description, tout oligomère comportant sur une seule extrémité, soit un groupe à insaturation éthylénique susceptible de polymériser par voie radicalaire avec les monomères constituant le squelette (S) du copolymère de l'invention et de se greffer sur la chaîne polymérique du squelette (S) ; soit un groupe fonctionnel réactif susceptible de réagir avec les monomères (A) et (B) du squelette (S) ou bien avec le squelette (S) préalablement formé.

Les macromonomères (M) greffés par liaison covalente sur la chaîne polymérique du squelette (S) des copolymères de l'invention, sont choisis préférentiellement parmi les macromonomères hydrocarbonés, hydrofluorocarbonés ou fluorocarbonés ayant une température de transition vitreuse T'g supérieure à 25°C.

Les macromonomères (M) ont une température de transition vitreuse T'g de préférence supérieure ou égale à 30°C.

Les macromonomères (M) sont de plus hydrophobes, c'est-à-dire insolubles dans l'eau et ont une tension de surface généralement inférieure ou égale à 40·10⁻⁵ N/cm (40 dyne/cm) à 20°C.

Ils présentent de préférence un poids moléculaire moyen mesuré en sommet de pic par chromatographie d'exclusion stérique allant de 200 à 30000.

Les macromonomères (M) de l'invention sont de préférence choisis dans le groupe consititué par:
- les oligomères copolymérisables, par voie radicalaire, avec les monomères du squelette (S) et présentant sur une des extrémités un radical terminal porteur d'une double liaison ;
- les oligomères présentant sur une des extrémités un radical terminal porteur d'une fonction réactive susceptible d'interagir avec le squelette (S) ou les monomères le constituant (telle que -OH, -NH₂,-COOH, anhydride) ;
- les oligomères polycondensables avec les monomères du squelette (S), présentant sur une des extrémités un radical terminal porteur d'une fonction réactive susceptible de participer à une polycondensation (telle que diol, diamine, diacide carboxylique).

Les macromonomères (M) de l'invention peuvent être aussi des oligomères polycondensats, par exemple, du type polyester, polyamide ou polyuréthane.

Parmi les macromonomères (M) greffés sur les copolymères de l'invention, on peut citer :
(a) les macromonomères de polystyrène de T'g supérieure à 25°C et présentant un groupe terminal à insaturation éthylénique (méthacrylate, acrylate, styrène, ...) ou une fonction réactive terminale susceptible d'interagir avec le squelette (S) ou les monomères le constituant ou une fonction réactive terminale susceptible de participer à une polycondensation (telle que diol, diamine, diacide carboxylique), parmi lesquels on peut citer en particulier ; les macromonomères de polystyrène et les macromonomères de polystyrène substitués à extrémité méthacrylate, dicarboxyle ou dihydroxyle tels que les produits vendus par la société TOA GOSEI ;
(b) les homopolymères et les copolymères d'acrylate ou de méthacrylate d'alkyle linéaire ou ramifié en C₁-C_{20,} de T'g supérieure à 25°C et présentant un groupe terminal choisi parmi vinyle, allyle, méthallyle, (méth)acryloyle, éthacryloyle, vinylbenzoyle, vinylbenzyle, alcényle en C₁-C₄, cycloalcényle en C₁-C₆, ou une fonction réactive terminale susceptible d'interagir avec le squelette (S) ou les monomères le constituant (telle que -OH, -NH₂, -COOH, anhydride) ou une fonction réactive terminale susceptible de participer à une polycondensation (telle que diol, diamine, diacide carboxylique), parmi lesquels on peut citer en particulier: les macromonomères de poly(méthacrylate de méthyle) à extrémité méthacrylate, dicarboxyle ou dihydroxyle tels que les produits vendus par la société TOA GOSEI ;
(c) les polymères ou copolymères de monomères fluorés ou perfluorés, de T'g supérieure à 25°C et présentant un groupe terminal à insaturation éthylénique ou une fonction réactive terminale susceptible d'interagir avec le squelette (S) ou les monomères le constituant ou une fonction réactive terminale susceptible de participer à une polycondensation, parmi lesquels on peut citer en particulier les homopolymères ou copolymères de (méth)acrylate de perfluoroalkyle.

Les macromonomères (M) sont présents dans la composition des copolymères de l'invention dans une proportion allant de préférence de 1 à 60% en poids par rapport au poids total du copolymère greffé.

Les copolymères greffés conformes à la présente invention, présentent de préférence un poids moléculaire moyen mesuré en sommet de pic par chromatographie d'exclusion stérique allant de 10 000 à 5 000 000.

Ils sont en général solubles ou dispersibles dans les milieux aqueux, les milieux alcooliques ou hydroalcooliques à base d'alcools inférieurs. Ils peuvent être non-ioniques, anioniques, cationiques ou amphotères ; les groupes ioniques étant situés préférentiellement dans la structure du squelette (S) pour apporter l'hydrophilie. Parmi les copolymères dispersibles, on peut citer les latex et les pseudo-latex.

Le squelette (S) des copolymères de l'invention ont une température de transition de phase Tg allant de 0 à 30°C.

Le squelette (S) des copolymères de l'invention est constitué d'un copolymère obtenu par polymérisation radicalaire ou par polycondensation.

Le squelette (S) obtenu par voie radicalaire, résulte, de préférence, de la polymérisation :
(a) d'au moins un monomère ou un mélange de monomères (A) à insaturation éthylénique, et éventuellement
(b) d'au moins un monomère ou un mélange de monomères (B) polaires et hydrophiles, à insaturation éthylénique ; les monomères (A) et (B) étant choisis de telle sorte que la température de transition de phase Tg du squelette (S) varie de 0 à 30°C. La présence du ou des monomères (B) dépendra de l'application cosmétique désirée.

Les monomères du type (A) sont choisis par exemple dans le groupe constitué par :
- les esters ou les amides acryliques ou méthacryliques obtenus à partir d'alcools aliphatiques, linéaires, ramifiés ou cycliques et/ou d'alcools aromatiques tels que le (méth)acrylate de méthyle, le (méth)acrylate d'éthyle, le (méth)acrylate de propyle, le (méth)acrylate de butyle, le (méth)acrylate d'isobutyle, le (méth)acrylate de tertio-butyle, le tertio-butyl acrylamide ;
- les esters ou amides vinyliques, allyliques ou méthallyliques obtenus à partir d'alcools aliphatiques, linéaires, ramifiés ou cycliques et/ou d'alcools aromatiques tels que l'acétate de vinyle, le propionate de vinyle, le benzoate de vinyle, le tertio-butyl benzoate de vinyle ;
- les oléfines tels que l'éthylène, le propylène, le styrène ou le styrène substitué ;
- les monomères acryliques ou vinyliques fluorés ou perfluorés ;
- leurs mélanges.

Les monomères du type (B) de l'invention sont choisis parmi les monomères hydrophiles et polaires à insaturation éthylénique anioniques, cationiques, amphotères ou non-ioniques ou leurs mélanges.

Parmi les monomères (B) anioniques, on peut citer :
- les monomères comportant au moins une fonction acide, sous forme libre ou bien sous forme partiellement ou totalement neutralisée tels que les mono acides carboxyliques comme les acides acrylique, méthacrylique, crotonique ; les diacides carboxyliques ou les anhydrides d'acide ainsi que leurs monoesters ou monoamides comme l'anhydride maléique sous forme de diacide, de monoester ou monoamide, l'acide itaconique ;
- les monomères comportant au moins une fonction acide sulfonique, sous forme libre ou bien sous forme partiellement ou totalement neutralisée tels que l'acide vinyl- ou styrène sulfonique, l'acide acrylamido-2 méthylpropane-2 sulfonique ;
- les monomères comportant au moins une fonction acide phosphorique ou phosphonique, sous forme libre ou bien sous forme partiellement ou totalement neutralisée.

Les monomères anioniques (B) sont de préférence partiellement ou totalement neutralisés par un composé monobasique telle qu'une base minérale comme la soude ou la potasse, ou un aminoalcool par exemple pris dans le groupe constitué par l'amino-2 méthyl-2 propanol-1 (AMP), la triéthanolamine, la triisopropanolamine (TIPA), la monoéthanolamine, la tri[(hydroxy-2) propyl-1 amine, l'amino-2 méthyl-2 propanediol-1,3 (AMPD), l'amino-2 hydroxyméthyl-2 propanediol-1,3.

Parmi les monomères (B) cationiques, on peut citer :
- les monomères comportant une fonction amine sous forme libre ou bien partiellement ou totalement neutralisée ou bien partiellement ou totalement quaternisée tels que le (méth)acrylate de diméthylaminoéthyle, le diméthylaminoéthyl méthacrylamide, la vinylamine, la vinylpyridine, le chlorure de diallyldiméthylammonium.

Les monomères cationiques (B) sont de préférence partiellement ou totalement neutralisés par un acide organique minéral ou organique tel que les acides chlorhydrique, acétique, lactique ou glycolique ou bien partiellement ou totalement quaternisés par un halogénure d'alkyle, de cycloalkyle, d'aryle ou un dialkylsulfate (diméthyl- ou diéthylsulfate).

Parmi les monomères (B) amphotères, on peut citer les carboxybétaïnes ou les sulfobétaïnes obtenues par quaternisation partielle ou totale de monomères à insaturation éthylénique comportant une fonction amine par des sels de sodium d'acide carboxylique à halogénure mobile (chloroacétate de sodium) ou par des sultones cycliques (propane sultone).

Parmi les monomères (B) non-ioniques, on peut citer :
- les (méth)acrylate d'hydroxyalkyle en C₁-C₄ tels que (méth)acrylate de 2-hydroxy éthyle, (méth)acrylate de 2-hydroxy propyle,
- les acrylamides tels que acrylamide, méthacrylamide, les dialkyl(C₁-C₄) (méth) acrylamides ;
- la N-vinylpyrrolidone ;
- les (méth)acrylates d'éthylèneglycol, de diéthylèneglycol, de polyéthylèneglycol à extrémité hydroxyle ou éther.

Le squelette (S) obtenu par polycondensation résulte, de préférence, de la réaction :
(a) d'au moins un monomère ou un mélange de monomères (A') polycondensable et éventuellement
(b) d'un monomère ou un mélange de monomères (B') polycondensable avec le ou les monomères (A'), portant au moins un groupe fonctionnel hydrophile apportant la solubilité ou la dispersibilité dans l'eau, les milieux alcooliques ou les milieux hydroalcooliques ; les monomères (A') et (B') étant choisis de telle sorte que la température de transition de phase Tg du squelette (S) varie de 0 à 30°C.

Les squelettes (S) du type polycondensat sont choisis par exemple parmi les polyesters, les polyamides, les polyuréthanes ou les polyesteramides comportant de préférence des groupes anioniques partiellement ou totalement neutralisés tels que carboxylique, sulfonique, des groupes cationiques partiellement ou totalement neutralisés ou quaternisés tels que des amines tertiaires. Ils peuvent également être de nature polyuréthane et/ou polyurée et comporter d'autres types de séquence telles que des polyethers et/ou polyesters et possédant des groupes ioniques, partiellement ou totalement neutralisés ou quaternisés.

Les copolymères greffés conformes à l'invention peuvent être obtenus par copolymérisation directe radicalaire de monomères (A) et (B) tels que définis ci-dessus constituant le squelette (S) et d'un macromonomère (M) présentant sur une seule extrémité un groupe à insaturation éthylénique copolymérisable avec les monomères (A) et (B).

La polymérisation directe radicalaire peut être faite alors en solution dans un solvant commun ou un mélange de solvants communs. Elle peut également être effectuée en milieu hétérogène, en particulier en suspension ou en émulsion dans l'eau ; le macromonomère étant dissous dans le mélange avec les monomères (A) et (B) tels que définis ci-dessus.

Lorsque le squelette (S) est un polycondensat tel qu'un polyester, un polyamide, un polyuréthane ou un polyesteramide, les copolymères greffés conformes à l'invention peuvent être obtenus par polycondensation directe de monomères (A') et (B') tels que définis ci-dessus constituant le squelette (S) et d'un macromonomère (M) présentant sur une seule extrémité deux fonctions réactives terminales (par exemple diol, diamine, diacide carboxylique, anhydride d'acide) susceptible de polycondenser avec les monomères (A') et (B').

La polycondensation directe peut être réalisée en solution, en dispersion ou en milieu fondu selon une réaction du type estérification, amidification, transestérification ou transamidification.

Enfin, les copolymères greffés conformes à l'invention peuvent être aussi obtenus en faisant réagir le copolymère du squelette (S), préalablement synthétisé, avec un macromonomère (M) présentant une fonction terminale réactive appropriée susceptible d'interagir avec le squelette (S) de préférence monofonctionnelle (amine, alcool, acide carboxylique, anhydride, époxy...). La réaction est généralement effectuée en solution ou dans un milieu fondu.

Les compositions cosmétiques et dermatologiques selon l'invention contiennent donc dans un support cosmétiquement acceptable les polymères tels que décrits ci-dessous, pour des applications aussi variées que celles rencontrées par exemple dans le domaine du capillaire, du maquillage ou bien encore des soins de la peau, ou de tout autre domaine cosmétique dans lequel l'utilisation d'une substance filmogène est désirable ou recherchée.

Les copolymères greffés selon l'invention peuvent être utilisés seuls comme agent filmogène ou bien comme additif à des agents filmogènes conventionnels dans et pour la préparation de compositions cosmétiques ou dermatologiques.

Parmi les applications préférentiellement visées par la présente invention, on peut plus particulièrement mentionner :
- le domaine des produits capillaires (lavage, soin ou beauté des cheveux), où les compositions selon l'invention, peuvent se présenter sous forme d'aérosols, de mousse, de shampooings, d'après-shampooings, de lotions ou de gels coiffants ou traitants, laques ou lotions de mise en forme ou de mise en plis ou encore de fixation.
- le domaine des produits de maquillage, en particulier pour le maquillage des ongles, des cils ou des lèvres, où les compositions selon l'invention peuvent se présenter sous forme de vernis à ongle ; de mascaras ou de eye-liners ; de rouges à lèvres.
- dans le domaine des produits de soin de la peau (crèmes, laits, lotions, masques, sérums, produits solaires).

La concentration en copolymère greffé dans les compositions cosmétiques ou dermatologiques de l'invention est généralement comprise entre 0,1 et 50%, et de préférence entre 1 et 30% en poids total de la composition. Elle varie selon l'application cosmétique ou dermatologique envisagée.

Dans le cas des vernis à ongles, cette proportion est en général supérieure ou égale à 30% en poids lorsque le copolymère de l'invention est utilisé seul comme agent filmogène.

Le support cosmétiquement acceptable des compositions selon l'invention est de préférence constitué d'eau, d'un ou plusieurs solvants organiques cosmétiquement acceptables ou bien d'un mélange d'eau et d'un ou plusieurs solvants organiques cosmétiquement acceptables.

Parmi ces solvants organiques, on utilise plus particulièrement les alcools inférieurs en C₁-C₄ tels que l'éthanol.

Les copolymères greffés selon l'invention sont dissous ou en dispersion dans le support des compositions de l'invention.

Les compositions peuvent en outre, et bien entendu, contenir divers adjuvants destinés à la rendre acceptable dans une application cosmétique particulière.

Les compositions selon l'invention peuvent contenir des additifs cosmétiques conventionnels choisis parmi les corps gras tels que les huiles minérales, végétales, animales ou de synthèse, les cires animales, fossiles, végétales, minérales ou de synthèse, des solvants organiques, des agents épaississants, des adoucissants, des agents anti-mousse, des agents hydratants, des humectants, des agents traitants (agents anti-chute, anti-pelliculaire, ...), des antiperspirants, des agents alcanisants, des filtres solaires UV-A ou UV-B ou à bande large, des colorants, des pigments, des parfums, des plastifiants, des conservateurs, des polymères organiques anioniques, non-ioniques ou amphotères compatibles avec les copolymères greffés de l'invention et des agents propulseurs lorsque les compositions se présentent sous forme aérosol.

Bien entendu, l'homme de l'art veillera à choisir le ou les éventuels composés complémentaires mentionnés ci-avant, de manière telle que les propriétés avantageuses attachées intrinsèquement aux compositions selon l'invention ne soient pas, ou substantiellement pas, altérées par la ou les adjonctions envisagées.

L'invention a également pour objet un procédé de traitement cosmétique des matières kératiniques telles que la peau, les cheveux, le cuir chevelu, les cils, les sourcils, les ongles, les lèvres, caractérisé en ce qu'il consiste à appliquer sur ces dernières une composition telle que définie ci-dessus.

### EXEMPLES

### EXEMPLE DE PREPARATION : Préparation d'un copolymère acrylique greffé par des greffons polystyrène

Le squelette (S) est un copolymère acide acrylique/acrylate d'isobutyle/acrylate de tertio-butyle. Le Macromonomère (M) utilisé est un oligomère polystyrène à extrémité mono-méthacrylate de masse moléculaire en nombre 6000 vendu sous le nom AS-6 par la Société TOA GOSEI CHEMICALS.

On réalise le copolymère greffé acrylique à partir de la composition suivante :
- Acrylate de tertio-butyle (A) 30% en poids
- Acrylate d'isobutyle (A) 50% en poids
- Acide acrylique (B) 10% en poids
- Macromonomère Polystyrène à extrémité mono-méthacrylate 10% en poids

Dans un réacteur avec agitation centrale, réfrigérant, thermomètre, barbotage d'azote, on introduit 100g du mélange de monomères décrit ci-dessus. On introduit ensuite 400g d'un mélange de solvants constitué par 320 g de tétrahydrofurane et 60g de toluène. On agite à 250 tours/minute et sous barbotage d'azote jusqu'à dissolution complète de tous les réactifs. On chauffe alors au reflux du mélange solvant.

On introduit ensuite 2 g d'amorceur tertio-butyl peroxy 2-éthyl hexanoate à 97% (vendu par la société AKZO sous le nom TRIGONOX 21 S) dès qu'on arrive au reflux. On maintient dans ces conditions d'agitation et de température pendant 12 heures. En fin de synthèse, on revient à la température ambiante, on dilue le milieu réactionnel par du tétrahydrofurane seul et on purifie le polymère par précipitation de la solution dans 5 1 d'éther de pétrole. On sèche le précipité sous vide à température de 50°C pendant 48 heures jusqu'à poids constant.

L'indice d'acide du polymère est de 80 (Théorique 77).

La caractérisation du poids moléculaire par chromatographie d'exclusion stérique sur colonnes de µ-styragel avec comme éluant du tétrahydrofurane vis à vis d'étalons polystyrène donne 1 seul pic principal correspondant à un poids moléculaire de 49.500. Aucune trace de macromonomère libre n'a été détecté.

La température de transition vitreuse Tg du squelette, mesurée par DSC (calorimétrie différentielle) est d'environ 10°C.

La température de transition vitreuse T'g du macromonomère, mesurée par DSC (calorimétrie différentielle) est d'environ 100°C.

### EXEMPLE DE FORMULATION : Laque de coiffage en aérosol

### Composition A :

- Copolymère acrylique greffé de l'exemple de préparation 8% en poids M.A. (M.A.matière active)
- Amino-2 méthyl-2 propanol-1 pour neutralisation à 100% qsp
- Ethanol qsp 100% en poids

La composition est dispersée dans l'éthanol à chaud. On obtient après retour à la température ambiante une dispersion laiteuse stable dans l'éthanol.

### Pressurisation :

- Composition A 70% en poids
- Diméthyléther 30% en poids

La laque aérosol obtenue, après application sur cheveux, apporte une bonne fixation de la chevelure avec une bonne facilité de démêlage sans effet collant au toucher et s'élimine facilement au shampooing.

## Revendications

1. Utilisation dans et pour la préparation de compositions cosmétiques ou dermatologiques, d'un copolymère greffé dont le squelette (S) est constitué d'un copolymère de température de transition vitreuse Tg allant de 0 à 30°C, susceptible d'être obtenu par polymérisation radicalaire ou par polycondensation et comportant sur la chaîne du squelette au moins un greffon constitué d'un macromonomère (M) hydrophobe et de température de transition vitreuse T'g supérieure à 25°C.

2. Utilisation selon la revendication 1, selon laquelle les macromonomères (M) greffés sont choisis parmi les macromonomères hydrophobes hydrocarbonés, hydrofluorocarbonés ou fluorocarbonés ayant une température de transition de phase T'g supérieure à 25°C.

3. Utilisation selon la revendication 1 ou 2, selon laquelle les macromonomères (M) greffés ont une température de transition de phase T'g supérieure ou égale à 30°C.

4. Utilisation selon l'une quelconque des revendications 1 à 3, selon laquelle les macromonomères (M) greffés ont une tension de surface généralement inférieure ou égale à 40·10⁻⁵ N/cm (40 dyne/cm) à 20°C.

5. Utilisation selon l'une quelconque des revendications 1 à 4, selon laquelle les macromonomères (M) greffés ont un poids moléculaire moyen, mesuré en sommet de pic par chromatographie d'exclusion stérique allant de 200 à 30000.

6. Utilisation selon l'une quelconque des revendications 1 à 5, selon laquelle les macromonomères (M) greffés sont choisis dans le groupe constitué par:
- les oligomères copolymérisables, par voie radicalaire, avec les monomères du squelette (S) et présentant sur une des extrémités un radical terminal porteur d'une double liaison ;
- les oligomères présentant sur une des extrémités un radical terminal porteur d'une fonction réactive terminale susceptible d'interagir avec le squelette (S) ou les monomères le constituant (telle que -OH, -NH₂,-COOH, anhydride) ;
- les oligomères polycondensables avec les monomères du squelette (S), présentant sur une des extrémités un radical terminal porteur d'une fonction réactive terminale susceptible de participer à une polycondensation (telle que diol, diamine, diacide carboxylique).

7. Utilisation selon la revendication 6, selon laquelle les macromères (M) son des oligomères polycondensats.

8. Utilisation selon la revendication 6, selon laquelle les macromères sont choisis dans le groupe constitué par:
(a) les macromonomères de polystyrène et les macromonomères de polystyrène substitués de T'g supérieure à 25°C et présentant un groupe terminal à insaturation éthylénique ou une fonction réactive terminale susceptible d'interagir avec le squelette (S) ou les monomères le constituant ou une fonction réactive terminale susceptible de participer à une polycondensation ;
(b) les homopolymères et les copolymères d'acrylate ou de méthacrylate d'alkyle linéaire ou ramifié en C₁-C_{20,} de T'g supérieure à 25°C et présentant un groupe terminal choisi parmi vinyle, allyle, méthallyle, (méth)acryloyle, éthacryloyle, vinylbenzoyle, vinylbenzyle, alcényle en C₁-C₄, cycloalcényle en C₁-C₆, ou une fonction réactive terminale susceptible d'interagir avec le squelette (S) ou les monomères le constituant ou une fonction réactive terminale susceptible de participer à une polycondensation ;
(c) les polymères ou copolymères de monomères fluorés ou perfluorés, de T'g supérieure à 25°C et présentant un groupe terminal à insaturation éthylénique ou une fonction réactive terminale susceptible d'interagir avec le squelette (S) ou les monomères le constituant ou une fonction réactive terminale susceptible de participer à une polycondensation.

9. Utilisation selon l'une quelconque des revendications 1 à 8, selon laquelle les macromonomères (M) greffés sont présents dans la composition des copolymères de l'invention dans une proportion allant de 1 à 60% en poids par rapport au poids total du copolymère greffé.

10. Utilisation selon l'une quelconque des revendications 1 à 9, caractérisée par le fait que le copolymère greffé présente un poids moléculaire moyen mesuré en sommet de pic par chromatographie d'exclusion stérique allant de 10 000 à 5 000 000.

11. Utilisation selon l'une quelconque des revendications 1 à 10, selon laquelle le squelette (S) est constitué d'un copolymère obtenu par polymérisation radicalaire :
(a) d'au moins un monomère ou un mélange de monomères (A) à insaturation éthylénique, et éventuellement
(b) d'au moins un monomère ou un mélange de monomères (B) polaires et hydrophiles, à insaturation éthylénique.

12. Utilisation selon l'une quelconque des revendications 1 à 10, selon laquelle le squelette (S) est constitué d'un copolymère obtenu par polycondensation :
(a) d'au moins un monomère ou un mélange de monomères (A') polycondensables et éventuellement
(b) d'un monomère ou un mélange de monomères (B') polycondensables avec le ou les monomères (A') et portant au moins un groupe fonctionnel hydrophile apportant la solubilité ou la dispersibilité dans l'eau, les milieux alcooliques ou les milieux hydroalcooliques ; les monomères (A') et (B') étant choisis de telle sorte que la température de transition de phase Tg du squelette (S) varie de 0 à 30°C.

13. Utilisation selon la revendication 11, le squelette (S) est un polycondensat choisi dans le groupe constitué par les polyesters, les polyamides, les polyuréthanes, les polyurées ou les polyesteramides comportant des groupes anioniques partiellement ou totalement neutralisés ou bien des groupes cationiques partiellement ou totalement neutralisés ou quaternisés ; les polycondensats multiséquencés du type polyuréthane et/ou polyurées et comportant d'autres types de séquence et possédant des groupes ioniques.

14. Utilisation selon la revendication 11, selon laquelle les monomères (A) sont choisis par exemple dans le groupe constitué par :
- les esters ou les amides acryliques ou méthacryliques obtenus à partir d'alcools aliphatiques, linéaires, ramifiés ou cycliques et/ou d'alcools aromatiques ;
- les esters ou amides vinyliques, allyliques ou méthallyliques obtenus à partir d'alcools aliphatiques, linéaires, ramifiés ou cycliques et/ou d'alcools aromatiques ;
- les oléfines ;
- les monomères acryliques ou vinyliques fluorés ou perfluorés ainsi que leurs mélanges.

15. Utilisation selon la revendication 11 ou 14, selon laquelle les monomères (A) sont choisis dans le groupe constitué par :
le (méth)acrylate de méthyle, le (méth)acrylate d'éthyle, le (méth)acrylate de propyle, le (méth)acrylate de butyle, le (méth)acrylate d'isobutyle, le (méth)acrylate de tertio-butyle, le tertio-butyl acrylamide, l'acétate de vinyle, le propionate de vinyle, le benzoate de vinyle, le tertio-butyl benzoate de vinyle, l'éthylène, le propylène, le styrène ou le styrène substitué ou leurs mélanges.

16. Utilisation selon l'une quelconque des revendications 11, 14 et 15, selon laquelle les monomères (B) sont choisis parmi les monomères à insaturation éthylénique, hydrophiles et polaires, anioniques, cationiques, amphotères ou non-ioniques ou leurs mélanges.

17. Utilisation selon la revendication 16, selon laquelle les monomères (B) anioniques sont choisis dans le groupe constitué par :
- les monomères comportant au moins une fonction acide, sous forme libre ou bien sous forme partiellement ou totalement neutralisée ;
- les monomères comportant au moins une fonction acide sulfonique, sous forme libre ou bien sous forme partiellement ou totalement neutralisée ;
- les monomères comportant au moins une fonction acide phosphorique ou phosphonique, sous forme libre ou bien sous forme partiellement ou totalement neutralisée.

18. Utilisation selon la revendication 17, selon laquelle les monomères (B) anioniques sont choisis parmi les mono acides carboxyliques ; les diacides carboxyliques ou les anhydrides d'acide ainsi que leurs monoesters ou monoamides ; l'acide vinyl- ou styrène sulfonique ; l'acide acrylamido-2 méthylpropane-2 sulfonique, lesdits monomères étant sous forme libre ou bien sous forme partiellement ou totalement neutralisée.

19. Utilisation selon la revendication 16, selon laquelle les monomères (B) cationiques sont choisis dans le groupe constitué par:
les monomères comportant au moins une fonction amine sous forme libre ou bien partiellement ou totalement neutralisée ou bien partiellement ou totalement quaternisée.

20. Utilisation selon la revendication 19, selon laquelle les monomères (B) cationiques sont choisis dans le groupe constitué par :
le (méth)acrylate de diméthylaminoéthyle, le diméthylaminoéthyl méthacrylamide, le vinylamine, la vinylpyridine, le chlorure de diallyldiméthylammonium, lesdits monomères étant sous forme libre ou bien sous forme partiellement ou totalement neutralisée ou bien sous forme partiellement ou totalement quaternisée.

21. Utilisation selon la revendication 16, selon laquelle les monomères (B) amphotères sont choisis dans le groupe constitué par :
les carboxybétaïnes ou les sulfobétaïnes obtenues par quaternisation partielle ou totale de monomères à insaturation éthylénique comportant une fonction amine par des sels de sodium d'acide carboxylique à halogénure mobile ou par des sultones cycliques.

22. Utilisation selon la revendication 16, selon laquelle les monomères (B) non-ioniques sont choisis dans le groupe constitué par:
- les (méth)acrylate d'hydroxyalkyle en C₁-C₄ ;
- les acrylamides ;
- la N-vinylpyrrolidone ;
- les (méth)acrylates d'éthylèneglycol, de diéthylèneglycol, de polyéthylèneglycol à extrémité hydroxyle ou éther.

23. Composition cosmétique ou dermatologique, caractérisée par le fait qu'elle contient dans un support cosmétiquement acceptable au moins un copolymère greffé tel que défini selon l'une quelconque des revendications 1 à 22.

24. Composition selon la revendication 23, caractérisée par le fait que la concentration en copolymère greffé dans les compositions cosmétiques est généralement comprise entre 0,1 et 50%, et de préférence entre 1 et 30% par rapport au poids total de la composition.

25. Composition selon la revendication 23 ou 24, caractérisée par le fait que le support cosmétiquement acceptable est de préférence constitué d'eau, d'un ou plusieurs solvants organiques cosmétiquement acceptables ou bien d'un mélange d'eau et d'un ou plusieurs solvants organiques cosmétiquement acceptables.

26. Composition selon la revendication 25, caractérisée par le fait que le ou les solvants organiques cosmétiquement acceptables sont choisis parmi les alcools inférieurs en C₁-C₄.

27. Composition selon l'une quelconque des revendications 23 à 26, caractérisée par le fait que le copolymère greffé est dissous ou en dispersion dans le support de la composition.

28. Composition selon l'une quelconque des revendications 23 à 27, caractérisée par le fait qu'elle contient des additifs cosmétiques conventionnels choisis parmi les corps gras tels que les huiles minérales, végétales, animales ou de synthèse, les cires animales, fossiles, végétales, minérales ou de synthèse, des solvants organiques, des agents épaississants, des adoucissants, des agents anti-mousse, des agents hydratants, des humectants, des agents traitants, des antiperspirants, des agents alcanisants, des agents acidifiants, des filtres solaires UV-A ou UV-B ou à bande large, des colorants, des pigments, des parfums, des plastifiants, des conservateurs, des polymères organiques anioniques, non-ioniques ou amphotères et les agents propulseurs

29. Composition selon l'une quelconque des revendications 23 à 28, caractérisée en ce qu'il s'agit d'une composition capillaire.

30. Composition selon l'une quelconque des revendications 23 à 29, caractérisée en ce qu'il s'agit d'une composition de maquillage.

31. Composition selon l'une quelconque des revendications 23 à 30, caractérisée en ce qu'il s'agit d'une composition pour le soin de la peau.

32. Utilisation d'un copolymère greffé tel que défini selon l'une quelconque des revendications 1 à 22 comme agent filmogène, ou comme additif d'agent filmogène, dans une et pour la préparation d'une composition cosmétique ou dermatologique.

33. Procédé de traitement cosmétique des matières kératiniques, caractérisé en ce qu'il consiste à appliquer sur ces dernières une composition cosmétique telle que définie à l'une quelconque des revendications 23 à 28.

## Patentansprüche

1. Verwendung in und für die Herstellung von kosmetischen oder dermatologischen Zusammensetzungen eines Pfropfcopolymers, dessen Hauptkette (H) aus einem Copolymer mit einer Glasübergangstemperatur Tg im Bereich von 0 bis 30 °C besteht, das durch radikalische Polymerisation oder Polykondensation hergestellt werden kann und das an der Hauptkette (H) mindestens einen Pfropfzweig trägt, der aus einem hydrophoben Makromonomer (M) mit einer Glasübergangstemperatur T'g oberhalb von 25 °C besteht.

2. Verwendung nach Anspruch 1, wobei die aufgepfropften Makromonomere (M) unter den hydrophoben Kohlenwasserstoff-, Fluorkohlenwasserstoff- und Fluorkohlenstoff-Makromonomeren ausgewählt werden, die eine Glasübergangstemperatur T'g oberhalb von 25 °C aufweisen.

3. Verwendung nach Anspruch 1 oder 2, wobei die aufgepfropften Makromonomere (M) eine Glasübergangstemperatur T'g aufweisen, die größer als oder gleich 30 °C ist.

4. Verwendung nach einem der Ansprüche 1 bis 3, wobei die aufgepfropften Makromonomere (M) eine Oberflächenspannung aufweisen, die im allgemeinen kleiner als oder gleich 40 · 10 ⁻⁵ N/cm (40 dyn/cm) bei 20 °C ist.

5. Verwendung nach einem der Ansprüche 1 bis 4, wobei die aufgepfropften Makromonomere (M) ein mittleres Molekulargewicht von 200 bis 30000 aufweisen, das durch sterische Ausschlußchromatographie an der Spitze des Peaks des Chromatogramms gemessen wird.

6. Verwendung nach einem der Ansprüche 1 bis 5, wobei die aufgepfropften Makromonomere (M) ausgewählt werden unter:
- Oligomeren, die radikalisch mit den Monomeren der Hauptkette (H) copolymerisierbar sind und an einem der Enden eine Endgruppe aufweisen, die eine Doppelbindung trägt;
- Oligomeren, die an einem der Enden eine Endgruppe aufweisen, die eine reaktive funktionelle Gruppe trägt, die mit der Hauptkette (H) oder den sie bildenden Monomeren zu reagieren vermag (wie z.B. -OH, -NH₂, -COOH, Anhydrid)
- Oligomeren, die mit den Monomeren der Hauptkette (H) polykondensierbar sind, die an einem der Enden eine Endgruppe aufweisen, die eine reaktive funktionelle Gruppe trägt, die an einer Polykondensationsreaktion teilzunehmen vermag (wie z.B. Diol, Diamin, Dicarbonsäure).

7. Verwendung nach Anspruch 6, wobei die Makromonomere (M) Polykondensat-Oligomere sind.

8. Verwendung nach Anspruch 6, wobei die Makromere (M) ausgewählt werden unter:
(a) Polystyrol-Makromonomeren und substituierten Polystyrol-Makromonomeren mit einer Glasübergangstemperatur T'g oberhalb von 25 °C, die eine ethylenisch ungesättigte endständige Gruppe oder eine endständige reaktive funktionelle Gruppe, die mit der Hauptkette (H) oder den die Hauptkette (H) bildenden Monomeren reagieren kann, oder eine endständige, reaktive funktionelle Gruppe, die an einer Polykondensationsreaktion teilzunehmen vermag, aufweisen,
(b) Alkylacrylat- und Alkylmethacrylat-Homopolymeren sowie Alkylacrylat- und Alkylmethacrylat-Copolymeren mit geradkettigem oder verzweigtem C₁-C₂₀-Alkyl mit einer Glasübergangstemperatur T'g oberhalb von 25 °C, die eine endständige Gruppe, die unter Vinyl, Allyl, Methallyl, (Meth)acryloyl, Ethacryloyl, Vinylbenzoyl, Vinylbenzyl, C₁-C₄-Alkenyl, C₁-C₆-Cycloalkenyl ausgewählt ist, oder eine endständige reaktive funktionelle Gruppe, die mit der Hauptkette (H) oder den die Hauptkette (H) bildenden Monomeren reagieren kann, oder eine endständige reaktive funktionelle Gruppe aufweisen, die an einer Polykondensationsreaktion teilzunehmen vermag,
(c) Polymeren und Copolymeren fluorhaltiger oder perfluorierter Monomere mit einer T'g oberhalb von 25 °C, die eine ethylenisch ungesättigte reaktive Endgruppe oder eine endständige reaktive funktionelle Gruppe, die mit der Hauptkette (H) oder den die Hauptkette (H) bildenden Monomeren zu reagieren vermag, oder eine endständige reaktive Gruppe, die an einer Polykondensationsreaktion teilzunehmen vermag, aufweisen.

9. Verwendung nach einem der Ansprüche 1 bis 8, wobei die aufgepfropften Makromonomere (M) in der Zusammensetzung der Copolymere in einem Anteil von 1 bis 60 Gew.-%, bezogen auf das Gesamtgewicht des Pfropfcopolymers, enthalten sind.

10. Verwendung nach einem der Ansprüche 1 bis 9, dadurch gekennzeichnet, daß das Pfropfcopolymer ein mittleres Molekulargewicht von 10 000 bis 5 000 000 aufweist, das durch sterische Ausschlußchromatographie an der Spitze des Peaks des Chromatogramms gemessen wird.

11. Verwendung nach einem der Ansprüche 1 bis 10, wobei die Hauptkette (H) aus einem Copolymer besteht, das durch radikalische Polymerisation
(a) mindestens eines ethylenisch ungesättigten Monomers (A) oder eines Gemischs derartiger Monomere (A) und gegebenenfalls
(b) mindestens eines polaren und hydrophilen, ethylenisch ungesättigten Monomers (B) oder eines Gemischs derartiger Monomere (B)
hergestellt wird.

12. Verwendung nach einem der Ansprüche 1 bis 10, wobei die Hauptkette (H) aus einem Copolymer besteht, das durch Polykondensation
(a) mindestens eines polykondensierbaren Monomers (A') oder eines Gemischs derartiger Monomere (A') und gegebenenfalls
(b) eines mit dem oder den Monomeren (A') polykondensierbaren Monomers (B') oder eines Gemischs derartiger Monomere (B'), die mindestens eine hydrophile funktionelle Gruppe tragen, die für die Löslichkeit oder Dispergierbarkeit in Wasser, alkoholischen oder wäßrig-alkoholischen Medien sorgt, wobei die Monomere (A') und (B') so ausgewählt werden, daß die Glasübergangstemperatur Tg der Hauptkette (H) im Bereich von 0 bis 30 °C liegt,
hergestellt wird.

13. Verwendung nach Anspruch 11, wobei die Hauptkette (H) ein Polykondensat ist, das ausgewählt wird unter Polyestern, Polyamiden, Polyurethanen, Polyharnstoffen und Polyesteramiden, die anionische Gruppen, die ganz oder teilweise neutralisiert sind, oder kationische Gruppen, die ganz oder teilweise neutralisiert oder quaternisiert sind, aufweisen, und multisequentiellen Polykondensaten vom Polyurethan- und/oder Polyharnstoff-Typ, die andere Sequenz-Typen enthalten und ionische Gruppen aufweisen.

14. Verwendung nach Anspruch 11, wobei die Monomere (A) beispielsweise ausgewählt werden unter:
- Acrylestern, Acrylamiden, Methacrylestern und Methacrylamiden, die mit aliphatischen geradkettigen, verzweigten oder cyclischen Alkoholen und/oder aromatischen Alkoholen hergestellt werden,
- Vinyl-, Allyl- und Methallylestern, Vinyl-, Allyl- und Methallylamiden, die mit aliphatischen geradkettigen, verzweigten oder cyclischen Alkoholen und/oder aromatischen Alkoholen hergestellt werden,
- Olefinen,
- fluorhaltigen oder perfluorierten Acrylmonomeren und fluorhaltigen oder perfluorierten Vinylmonomeren,
- sowie ihren Gemischen.

15. Verwendung nach Anspruch 11 oder 14, wobei die Monomere (A) ausgewählt werden unter:
Methyl(meth)acrylat, Ethyl(meth)acrylat, Propyl(meth)acrylat, Butyl(meth)acrylat, Isobutyl(meth)acrylat, *tert.*-Butyl(meth)acrylat, *tert.*-Butylacrylamid, Vinylacetat, Vinylpropionat, Vinylbenzoat, Vinyl-*tert*.-butylbenzoat, Ethylen, Propylen, Styrol, substituiertem Styrol und ihren Gemischen.

16. Verwendung nach einem der Ansprüche 11, 14 und 15, wobei die Monomere (B) unter anionischen, kationischen, amphoteren und nichtionischen, hydrophilen und polaren, ethylenisch ungesättigten Monomeren und ihren Gemischen ausgewählt werden.

17. Verwendung nach Anspruch 16, wobei die anionischen Monomere (B) ausgewählt werden unter:
- Monomeren, die mindestens eine Säuregruppe aufweisen, die in freier Form vorliegt oder ganz oder teilweise neutralisiert ist,
- Monomeren, die mindestens eine Sulfonsäuregruppe aufweisen, die in freier Form vorliegt oder ganz oder teilweise neutralisiert ist,
- Monomeren, die mindestens eine Phosphorsäure- oder Phosphonsäuregruppe aufweisen, die in freier Form vorliegt oder ganz oder teilweise neutralisiert ist.

18. Verwendung nach Anspruch 17, wobei die anionischen Monomere (B) unter Monocarbonsäuren, Dicarbonsäuren und Säureanhydriden sowie ihren Monoestern oder Monoamiden, Vinylsulfonsäure und Styrolsulfonsäure, 2-Acrylamido-2-methylpropansulfonsäure ausgewählt werden, wobei die Monomere in freier Form vorliegen oder ganz oder teilweise neutralisiert sind.

19. Verwendung nach Anspruch 16, wobei die kationischen Monomere (B) unter den Monomeren ausgewählt werden, die mindestens eine Amingruppe enthalten, die in freier Form vorliegt oder ganz oder teilweise neutralisiert oder ganz oder teilweise quaternisiert ist.

20. Verwendung nach Anspruch 19, wobei die kationischen Monomere (B) unter Dimethylaminoethyl(meth)acrylat, Dimethylaminoethylmethacrylamid, Vinylamin, Vinylpyridin, Diallyldimethylammoniumchlorid ausgewählt werden, wobei die Monomere in freier Form vorliegen oder ganz oder teilweise neutralisiert sind oder ganz oder teilweise quaternisiert sind.

21. Verwendung nach Anspruch 16, wobei die amphoteren Monomere (B) unter Carboxybetainen und Sulfobetainen ausgewählt werden, die durch vollständige oder teilweise Quaternisierung von ethylenisch ungesättigten Monomeren, die eine Aminogruppe aufweisen, mit Natriumsalzen von Carbonsäuren, die bewegliches Halogen enthalten, oder mit cyclischen Sultonen hergestellt werden.

22. Verwendung nach Anspruch 16, wobei die nichtionischen Monomere (B) ausgewählt werden unter:
- C₁-C₄-Hydroxyalkyl (meth)acrylaten,
- Acrylamiden,
- N-Vinylpyrrolidon,
- Ethylenglykol-, Diethylenglykol-, Polyethylenglykol(meth)acrylaten mit Hydroxy- oder Ether-Endgruppe.

23. Kosmetische oder dermatologische Zusammensetzung, dadurch gekennzeichnet, daß sie in einem kosmetisch akzeptablen Träger mindestens ein Pfropfcopolymer enthält, das wie in einem der Ansprüche 1 bis 22 definiert ist.

24. Zusammensetzung nach Anspruch 23, dadurch gekennzeichnet, daß die Konzentration an Pfropfcopolymer in den kosmetischen Zusammensetzungen im allgemeinen im Bereich von 0,1 bis 50 Gew.-% und vorzugsweise 1 bis 30 Gew.-%, bezogen auf das Gesamtgewicht der Zusammensetzung, liegt.

25. Zusammensetzung nach Anspruch 23 oder 24, dadurch gekennzeichnet, daß der kosmetisch akzeptable Träger vorzugsweise aus Wasser, einem oder mehreren kosmetisch akzeptablen organischen Lösungsmitteln oder einem Gemisch aus Wasser und einem oder mehreren kosmetisch akzeptablen organischen Lösungsmitteln besteht.

26. Zusammensetzung nach Anspruch 25, dadurch gekennzeichnet, daß das oder die kosmetisch akzeptablen organischen Lösungsmittel unter niederen C₁-C₄-Alkoholen ausgewählt sind.

27. Zusammensetzung nach einem der Ansprüche 23 bis 26, dadurch gekennzeichnet, daß das Pfropfcopolymer in dem Träger der Zusammensetzung gelöst oder dispergiert ist.

28. Zusammensetzung nach einem der Ansprüche 23 bis 27, dadurch gekennzeichnet, daß sie herkömmliche kosmetische Zusatzstoffe enthält, die ausgewählt sind unter Fettsubstanzen, wie z.B. anorganischen, pflanzlichen, tierischen und synthetischen Ölen, tierischen Wachsen, Erdölwachsen, anorganischen und synthetischen Wachsen, organischen Lösungsmitteln, Verdikkungsmitteln, reizlindernden Mitteln, Antischaummitteln, Hydratisierungsmitteln, Feuchthaltemitteln, der Behandlung dienenden Mitteln, Antiperspirantien, alkalisch machenden Mitteln, sauer machenden Mitteln, UV-A-, UV-B- und Breitband-Sonnenschutzfiltern, Farbstoffen, Pigmenten, Parfums, Weichmachern, Konservierungsmitteln, anionischen, nichtionischen und amphoteren organischen Polymeren und Treibmitteln.

29. Zusammensetzung nach einem der Ansprüche 23 bis 28, dadurch gekennzeichnet, daß es sich um eine Zusammensetzung für die Haarbehandlung handelt.

30. Zusammensetzung nach einem der Ansprüche 23 bis 29, dadurch gekennzeichnet, daß es sich um eine Schminkzusammensetzung handelt.

31. Zusammensetzung nach einem der Ansprüche 23 bis 30, dadurch gekennzeichnet, daß es sich um eine Zusammensetzung für die Hautpflege handelt.

32. Verwendung eines Pfropfcopolymers, das wie in einem der Ansprüche 1 bis 22 definiert ist, als Filmbildner oder als Zusatz zu Filmbildnern in und für die Herstellung von kosmetischen oder dermatologischen Zusammensetzungen.

33. Verfahren zur kosmetischen Behandlung von Keratinmaterialien, dadurch gekennzeichnet, daß es darin besteht, auf die Keratinmaterialien eine wie in einem der Ansprüche 23 bis 28 definierte Zusammensetzung aufzutragen.

## Claims

1. Use in and for the preparation of cosmetic or dermatological compositions of a graft copolymer whose backbone (S) consists of a copolymer with a glass transition temperature Tg ranging from 0 to 30°C, capable of being obtained by radical polymerization or by polycondensation and comprising on the backbone chain at least one graft unit consisting of a hydrophobic macromonomer (M) with a glass transition temperature T'g higher than 25°C.

2. Use according to Claim 1, according to which the grafted macromonomers (M) are chosen from hydrocarbon, hydrofluorocarbon or fluorocarbon hydrophobic macromonomers which have a phase transition temperature T'g higher than 25°C.

3. Use according to Claim 1 or 2, according to which the grafted macromonomers (M) have a phase transition temperature T'g higher than or equal to 30°C.

4. Use according to any one of Claims 1 to 3, according to which the grafted macromonomers (M) have a surface tension generally lower than or equal to 40 × 10⁻⁵N/cm (40 dyne/cm) at 20°C.

5. Use according to any one of Claims 1 to 4, according to which the grafted macromonomers (M) have a mean molecular weight, measured at the top of a peak using steric exclusion chromatography, ranging from 200 to 30 000.

6. Use according to any one of Claims 1 to 5, according to which the grafted macromonomers (M) are chosen from the group consisting of:
- oligomers which can copolymerize by a radical route with the monomers of the backbone (S) and which have on one of the ends an end radical carrying a double bond;
- oligomers which have on one of the ends an end radical carrying a reactive functional end group capable of interacting with the backbone (S) or the monomers of which it consists (such as -OH, -NH₂, -COOH or anhydride);
- oligomers which can polycondense with the monomers of the backbone (S), which have on one of the ends an end radical carrying a reactive functional end group capable of taking part in a polycondensation (such as a diol, diamine or dicarboxylic acid).

7. Use according to Claim 6, according to which the macromers (M) are polycondensate oligomers.

8. Use according to Claim 6, according to which the macromers are chosen from the group consisting of:
(a) polystyrene macromonomers and substituted polystyrene macromonomers of T'g higher than 25°C and which have an end group containing ethylenic unsaturation or a reactive functional end group capable of interacting with the backbone (S) or the monomers of which it consists or a reactive functional end group capable of taking part in a polycondensation;
(b) linear or branched C₁-C₂₀ alkyl acrylate or methacrylate homopolymers and copolymers of T'g higher than 25°C and which have an end group chosen from vinyl, allyl, methallyl, (meth)acryloyl, ethacryloyl, vinylbenzoyl, vinylbenzyl, C₁-C₄ alkenyl and C₁-C₆ cycloalkenyl or a reactive functional end group capable of interacting with the backbone (S) or the monomers of which it consists or a reactive functional end group capable of taking part in a polycondensation;
(c) polymers or copolymers of fluorinated or perfluorinated monomers, of T'g higher than 25°C and which have an end group containing ethylenic unsaturation or a reactive functional end group capable of interacting with the backbone (S) or the monomers of which it consists or a reactive functional end group capable of taking part in a polycondensation.

9. Use according to any one of Claims 1 to 8, in which the grafted macromonomers (M) are present in the composition of the copolymers of the invention in a proportion ranging from 1 to 60 % by weight relative to the total weight of the graft copolymer.

10. Use according to any one of Claims 1 to 9, characterized in that the graft copolymer has a mean molecular weight, measured at the top of a peak using steric exclusion chromatography, ranging from 10 000 to 5 000 000.

11. Use according to any one of Claims 1 to 10, according to which the backbone (S) consists of a copolymer obtained by radical polymerization:
(a) of at least one monomer or mixture of monomers (A) containing ethylenic unsaturation, and optionally
(b) of at least one polar and hydrophilic monomer or mixture of monomers (B) containing ethylenic unsaturation.

12. Use according to any one of Claims 1 to 10, according to which the backbone (S) consists of a copolymer obtained by polycondensation:
(a) of at least one polycondensable monomer or mixture of monomers (A') and optionally
(b) of a monomer or mixture of monomers (B') which can polycondense with the monomer(s) (A') and carrying at least one hydrophilic functional group contributing solubility or dispersibility in water, alcoholic media or hydroalcoholic media, the monomers (A') and (B') being chosen such that the phase transition temperature Tg of the backbone (S) varies from 0 to 30°C.

13. Use according to Claim 11, according to which the backbone (S) is a polycondensate chosen from the group consisting of polyesters, polyamides, polyurethanes, polyureas or polyesteramides containing partially or completely neutralized anionic groups or else partially or completely neutralized or quaternized cationic groups, multiblock polycondensates of the polyurethane and/or polyurea type and containing other types of block and possessing ionic groups.

14. Use according to Claim 11, according to which the monomers (A) are chosen, for example, from the group consisting of:
- acrylic or methacrylic esters or amides obtained from linear, branched or cyclic aliphatic alcohols and/or aromatic alcohols;
- vinyl, allyl or methallyl esters or amides obtained from linear, branched or cyclic aliphatic alcohols and/or aromatic alcohols;
- olefins;
- fluorinated or perfluorinated acrylic or vinyl monomers and mixtures thereof.

15. Use according to Claim 11 or 14, according to which the monomers (A) are chosen from the group consisting of:
methyl (meth)acrylate, ethyl (meth)acrylate, propyl (meth)acrylate, butyl (meth)acrylate, isobutyl (meth)acrylate, tert-butyl (meth)acrylate, tert-butylacrylamide, vinyl acetate, vinyl propionate, vinyl benzoate, vinyl tert-butylbenzoate, ethylene, propylene, styrene and substituted styrene and mixtures thereof.

16. Use according to any one of Claims 11, 14 and 15, according to which the monomers (B) are chosen from hydrophilic and polar, anionic, cationic, amphoteric or nonionic monomers containing ethylenic unsaturation and mixtures thereof.

17. Use according to Claim 16, according to which the anionic monomers (B) are chosen from the group consisting of:
- monomers containing at least one acidic functional group in free form or else in a partially or completely neutralized form;
- monomers containing at least one sulphonic acid functional group in free form or else in a partially or completely neutralized form;
- monomers containing at least one phosphoric or phosphonic acid functional group in free form or else in a partially or completely neutralized form.

18. Use according to Claim 17, according to which the anionic monomers (B) are chosen from monocarboxylic acids, dicarboxylic acids or acid anhydrides and their monoesters or monoamides, vinyl- or styrenesulphonic acid and 2-acrylamido-2-methylpropanesulphonic acid, the said monomers being in free form or else in a partially or completely neutralized form.

19. Use according to Claim 16, according to which the cationic monomers (B) are chosen from the group consisting of:
monomers containing at least one amine functional group in free form or else partially or completely neutralized or else partially or completely quaternized.

20. Use according to Claim 19, according to which the cationic monomers (B) are chosen from the group consisting of:
dimethylaminoethyl (meth)acrylate, dimethylaminoethylmethacrylamide, vinylamine, vinylpyridine and diallyldimethylammonium chloride, the said monomers being in free form or else in a partially or completely neutralized form or else in a partially or completely quaternized form.

21. Use according to Claim 16, according to which the amphoteric monomers (B) are chosen from the group consisting of:
carboxybetaines or sulphobetaines obtained by partial or complete quaternization of monomers containing ethylenic unsaturation comprising an amine functional group with sodium salts of a carboxylic acid containing labile halide or with cyclic sultones.

22. Use according to Claim 16, according to which the nonionic monomers (B) are chosen from the group consisting of:
- C₁-C₄ hydroxyalkyl (meth)acrylates;
- acrylamides;
- N-vinylpyrrolidone;
- ethylene glycol, diethylene glycol and polyethylene glycol (meth)acrylates containing a hydroxyl or ether end.

23. Cosmetic or dermatological composition characterized in that it contains at least one graft copolymer as defined according to any one of Claims 1 to 22 in a cosmetically acceptable carrier.

24. Composition according to Claim 23, characterized in that the concentration of graft copolymer in the cosmetic compositions is generally between 0.1 and 50 % and preferably between 1 and 30 %, relative to the total weight of the composition.

25. Composition according to Claim 23 or 24, characterized in that the cosmetically acceptable carrier preferably consists of water, of one or several cosmetically acceptable organic solvents or else of a mixture of water and of one or several cosmetically acceptable organic solvents.

26. Composition according to Claim 25, characterized in that the cosmetically acceptable organic solvent(s) are chosen from C₁-C₄ lower alcohols.

27. Composition according to any one of Claims 23 to 26, characterized in that the graft copolymer is dissolved or in dispersion in the carrier of the composition.

28. Composition according to any one of Claims 23 to 27, characterized in that it contains conventional cosmetic additives chosen from fatty substances such as mineral, vegetable, animal or synthetic oils, animal, fossil, vegetable, mineral or synthetic waxes, organic solvents, thickening agents, emollients, antifoam agents, hydrating agents, moisteners, treating agents, antiperspirants, alkalifying agents, acidifying agents, UV-A or UV-B or broad band sunscreens, dyes, pigments, perfumes, plasticizers, preserving agents, anionic, nonionic or amphoteric organic polymers and propellent agents.

29. Composition according to any one of Claims 23 to 28, characterized in that it is a hair-care composition.

30. Composition according to any one of Claims 23 to 29, characterized in that it is a make-up composition.

31. Composition according to any one of Claims 23 to 30, characterized in that it is a composition for skin care.

32. Use of a graft copolymer as defined according to any one of Claims 1 to 22 as film-forming agent or as film-forming agent additive in and for the preparation of a cosmetic or dermatological composition.

33. Process for the treatment of keratinous materials, characterized in that it consists in applying to the latter a cosmetic composition as defined in any one of Claims 23 to 28.
